Europäisches Patentamt

European Patent Office

Office européen des brevets

(19)

(11) Publication number: **0 080 862**
**B1**

(12) **EUROPEAN PATENT SPECIFICATION**

(45) Date of publication of patent specification: **25.09.85**

(21) Application number: **82306274.0**

(22) Date of filing: **25.11.82**

(51) Int. Cl.⁴: **A 61 K 31/43,** A 61 K 9/00 // (A61K31/43, 31:42)

(54) Pharmaceutical formulation comprising beta-lactam antibiotics.

(30) Priority: **02.12.81 GB 8136295**

(43) Date of publication of application:
**08.06.83 Bulletin 83/23**

(45) Publication of the grant of the patent:
**25.09.85 Bulletin 85/39**

(84) Designated Contracting States:
**BE CH DE FR GB IT LI NL SE**

(56) References cited:
**EP-A-0 010 904**
**EP-A-0 049 061**
**GB-A-1 532 993**
**GB-A-2 005 538**

(73) Proprietor: **BEECHAM GROUP PLC**
**Beecham House Great West Road**
**Brentford Middlesex TW8 9BD (GB)**

(72) Inventor: **Grimmett, Francis Walter**
**12 Hobbs Way**
**Rustington Sussex (GB)**
Inventor: **Burnstead, Harry William**
**Burnstead, Barry William**
**Worthing Sussex (GB)**
Inventor: **Hartnell, Michael William**
**18 Tavy Close Durrington**
**Worthing West Sussex (GB)**

(74) Representative: **Hesketh, Alan, Dr. et al**
**European Patent Attorney Beecham**
**Pharmaceuticals Great Burgh Yew Tree Bottom**
**Road**
**Epsom, Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## 0 080 862

**Description**

The present invention relates to pharmaceutical compositions for oral administration in the treatment of bacterial infections.

West German OLS No. 28 43 318 discloses pharmaceutical compositions suitable for oral administration which comprise 20 mg to 1500 mg of amoxycillin trihydrate, 20 mg to 500 mg of potassium clavulanate and a pharmaceutically acceptable carrier with the proviso that the weight ratio of amoxycillin trihydrate to potassium clavulanate is from 6:1 to 1:1.

It has now been found that pharmaceutical compositions comprising amoxycillin trihydrate and potassium clavulanate may be prepared in the form of an extrudate suitable for direct ingestion or dispersion in water prior to ingestion.

Accordingly, the present invention provides a water-dispersible composition comprising amoxycillin trihydrate and potassium clavulanate and conventional excipients characterized in that it contains a non-hygroscopic water-soluble binder in an amount sufficient to render said composition extrudable after addition of a solvent for the binder.

Suitably the weight ratio of amoxycillin to clavulanic acid in the pharmaceutical composition of the present invention is from 12:1 to 2:1, for example 8:1, 6:1, 5:1, 4:1, 3:1 or 2:1. Preferred weight ratios of amoxycillin to clavulanic acid are 8:1, 4:1 and 2:1.

Suitable conventional excipients include flavouring agents, preservatives and colouring agents. The materials present in such composition should have low free moisture contents and preferably be pre-dried; advantageously an edible desiccant may be incorporated in the composition. Tightly bound water, such as water of crystallisation normally has little adverse effect on stability.

The water soluble non-hygroscopic binder is suitably soluble in organic solvent and is suitably an organic polymer. Preferred water soluble non-hygroscopic binders include hydroxypropylmethylcellulose and polyvinylpyrrolidone/polyvinylacetate copolymer, and hydroxypropylcellulose.

The organic solvent in which the non-hygroscopic binder is soluble may be any pharmaceutically acceptable inert solvent for the binder which is sufficiently volatile to be readily removed from the extrudate. Examples of such solvents include methanol, ethanol, *n*- and *iso*-propanol, chloroform, methylene chloride, acetone, methylethylketone, methyl acetate, ethyl acetate, trichloroethylene, tetrachloroethylene, carbon tetrachloride or homogeneous mixtures of these solvents.

Also included within the scope of the present invention is a process for the preparation of an extrudable water-dispersible composition as hereinbefore described which comprises bringing into association the components of said composition and thereafter extruding the blended mixture.

It is preferable that the formulation of the composition is carried out in a dry atmosphere, e.g. one containing less than 40% relative humidity and preferably one containing less than 30% relative humidity.

After the preparation of the extrudate it will normally be sieved to remove any particles present of undesirable proportions. Optionally an edible desiccant material may be dusted onto the extrudate.

The extrudate may be administered orally either in the dry form, or as a reconstituted syrup. The syrup may be formed from the extrudate in the usual way, for example by agitating the extrudate in a suitable solvent, such as water, together with conventional syrup additives if so desired. The composition may be presented for example in protective packages such as screw cap bottles or aluminium foil sachets containing multiple doses, or in single dose sachets.

The weight of extrudate in a single dose will depend on factors such as the actual ratio of amoxycillin trihydrate and potassium clavulanate used, and the nature of the bacterial infection being treated, and will be a weight suitable for the intended mode of administration. Normally, for example, between 0.25—10 g more suitably 0.5—5 g of the extrudate will comprise a single dose.

The weight of amoxycillin trihydrate and potassium clavulanate in a single dose of the extrudate will be such that the single dose contains sufficient amoxycillin trihydrate and potassium clavulanate for effective treatment of the infection. The single dose will be repeated according to the usual dosage regime for amoxycillin trihydrate and potassium clavulanate compositions.

In the following Examples, which illustrate the invention, the compositions were formulated under a dry atmosphere.

Example 1
Extrude granules of the following composition were prepared:

| Ingredients | mg per unit dose | % |
|---|---|---|
| Amoxycillin trihydrate BP equivalent to amoxycillin | 136.50 | 13.650 |
| Potassium clavulanate equivalent to clavulanic acid | 70.00 | 7.000 |
| Silicic acid, anhydrous (Syloid® ALI) | 50.00 | 5.000 |
| Hydroxypropylmethyl cellulose (Pharmacoat®603) | 40.00 | 4.000 |
| Flavour | 13.00 | 1.300 |
| Monoammonium glycyrrhizinate | 1.00 | 0.100 |
| Colouring | 0.50 | 0.050 |
| Sugar (milled) to | 1000.00 | 100.000 |

Syloid®ALI is supplied by W. R. Grace Ltd.
Pharmacoat®603 is supplied by Shinetsu Chemical Co.

The granules were prepared by passing the ingredients and the Pharmacoat®603 except the Syloid® through a 500 μm (30 mesh) British Standard stainless steel screen and then blending them in a Planetary mixer. The dry powder mix was then stirred at slow speed and dichloromethane added to form a granular mass. The granulated mass then passed through a Fuji Paudal Radial extruder, the extruded product collected, passed through a 1000 μm (16 mesh) screen and dried for 35 minutes at less than 45°C. The dried extrudates were blended with 5% Syloid® and stored in sealed containers containing molecular sieve desiccant.

Example 2
Extruded granules of the following composition were prepared:

| Ingredients | mg/per Unit dose | % w/w |
|---|---|---|
| Amoxycillin trihydrate B.P. equiv. to amoxycillin | 102.5 | 10.25 |
| Potassium clavulanate equiv. to clavulanic acid | 52.5 | 5.25 |
| Silicic acid, anhydrous (Syloid®ALI) | 50.0 | 5.0 |
| Hydroxypropylmethyl cellulose (Pharmacoat®603) | 40.0 | 4.0 |
| Flavour | 11.2 | 1.12 |
| Colouring | 0.5 | 0.05 |
| Sugar (milled) to | 1000 | 100.00 |

The granules were prepared by passing the ingredients except the Syloid®ALI through the 30# screen, and blending in a planetary mixer. The granulation and drying were carried out as in Example 1, and the Syloid® dusted into the final product before putting into suitable containers.

Example 3

The ingredients and process were as for Example 2, but the Syloid® was mixed into the wet mass after granulation, but before extrusion and drying.

Example 4

The ingredients were as for Example 2, but the Pharmacoat® 603 level was reduced to 1% w/w. All ingredients except the Syloid® were screened and blended as in Example 1, and then the powder wetted with dichloromethane to form a workable wet mass. This was extruded and dried as in Example 1, and the Syloid® dusted onto the final product.

Example 5

The Pharmacoat®603 in Example 1 was replaced with Kollidon®VA64 (r.t.m. B.A.S.F.), a polyvinylpyrrolidone/polyvinylacetate copolymer, at the 10% w/w level, dissolved in a 1:1 iso-propanol/dichloromethane solvent. This solution was used for the granulation step.

Example 6

The ingredients and process were as for examples 1 and 2, except that the Pharmacoat® was excluded from the dry mixing stage. The Pharmacoat® was dissolved in a mix of isopropylalcohol and dichloromethane and used to wet mass the dry mix.

Example 7

The ingredients and screening of ingredients were as for Example 1 but the mixing and extruding processes were carried out using a Winkworth Z-Blade Extruder.

The extrudate was then dried as in Example 1, and the Syloid® dusted onto the final product.

**Claims**

1. A water-dispersible composition comprising amoxycillin trihydrate and potassium clavulanate and conventional excipients, characterized in that it contains a non-hygroscopic water-soluble binder in an amount sufficient to render said composition extrudable after addition of a solvent for the binder.

2. A composition as claimed in claim 1 characterized in that the weight ratio of amoxycillin to clavulanic acid is from 12:1 to 2:1.

3. A composition as claimed in claim 1 or claim 2 characterized in that the weight ratio of amoxycillin to clavulanic acid is 8:1, 4:1 or 2:1.

4. A composition as claimed in any of claims 1 to 3 characterized in that the water soluble non-hygroscopic binder is an organic polymer.

5. A composition as claimed in any of claims 1 to 4 characterized in that the water soluble non-hygroscopic binder is hydroxypropylmethylcellulose, polyvinylpyrrolidone/polyvinylacetate copolymer or hydroxypropylcellulose.

6. An extrudate characterized in that it is obtained from a composition as claimed in any of Claims 1—5.

7. An extrudate as claimed in Claim 6 characterized in that the weight of extrudate in a single dose is between 0.25 g and 10 g.

8. An extrudate as claimed in Claim 6 characterized in that the weight of extrudate in a single dose is between 0.5 and 5 g.

9. A process for the preparation of an extrudate as claimed in any of claims 6 to 8, which process is characterized in that it comprises bringing into association amoxycillin trihydrate, potassium clavulanate, a non-hygroscopic water-soluble binder, and conventional excipients in a solvent for the said binder, and thereafter extruding the blended mixture.

10. A composition for use in treatment of bacterial infections in man, characterized in that it consists of amoxycillin trihydrate and potassium clavulanate in the form of an extrudate claimed in any of Claims 6 to 8.

**Patentansprüche**

1. Eine in Wasser dispergierbare Zusammensetzung enthaltend Amoxycillintrihydrat und Kaliumclavulanat und herkömmliche Trägerstoffe, dadurch gekennzeichnet, daß sie ein nichthygroskopisches wasserlösliches Bindemittel enthält, und zwar in ausreichender Menge, um die genannte Zusammensetzung nach Zugabe eines Lösungsmittels für das Bindemittel extrudierbar zu machen.

2. Eine Zusammensetzung wie in Anspruch 1 beansprucht, dadurch gekennzeichnet, daß das Gewichtsverhältnis von Amoxycillin zu Clavulansäure im Bereich zwischen 12:1 und 2:1 liegt.

3. Eine Zusammensetzung wie in Anspruch 1 oder 2 beansprucht, dadurch gekennzeichnet, daß das Gewichtsverhältnis von Amoxycillin zu Clavulansäure 8:1, 4:1 oder 2:1 beträgt.

4. Eine Zusammensetzung wie in einem der Ansprüche 1 bis 3 beansprucht, dadurch gekennzeichnet, daß das wasserlösliche nicht-hygroskopische Bindemittel ein organisches Polymer ist.

5. Eine Zusammensetzung wie in einem der Ansprüche 1 bis 4 beansprucht, dadurch gekennzeichnet,

4

daß das wasserlösliche nicht-hygroskopische Bindemittel Hydroxypropylmethylcellulose, Polyvinylpyrrolidon-Polyvinylacetat-Copolymer oder Hydroxypropylcellulose ist.

6. Ein Extrudat dadurch gekennzeichnet, daß es aus einer Zusammensetzung wie in den Ansprüchen 1 bis 5 beansprucht erhalten wird.

7. Ein Extrudat wie in Anspruch 6 beansprucht, dadurch gekennzeichnet, daß das Gewicht des Extrudats in einer Einzeldosis zwischen 0,25 und 10 g beträgt.

8. Ein Extrudat wie in Anspruch 6 beansprucht, dadurch gekennzeichnet, daß das Gewicht des Extrudats in einer Einzeldosis zwischen 0,5 und 5 g beträgt.

9. Ein Verfahren zur Herstellung eines Extrudats wie in einem der Ansprüche 6 bis 8 beansprucht, welches Verfahren dadurch gekennzeichnet ist, daß es das Zusammenbringen von Amoxycillintrihydrat, Kaliumclavulanat, einem nicht-hygroskopischen wasserlöslichen Bindemittel und herkömmlichen Trägersubstanzen in einem Lösungsmittel für das Bindemittel und anschließend das Extrudieren dieser so gebildeten Mischung umfaßt.

10. Eine Zusammensetzung zur Verwendung bei der Behandlung von bakteriellen Infektionen beim Menschen, dadurch gekennzeichnet, daß sie aus Amoxycillintrihydrat und Kaliumclavulanat in Form eines Extrudats wie in einem der Ansprüche 6 bis 8, beansprucht, besteht.

**Revendications**

1. Composition pouvant se disperser dans l'eau comprenant de l'amoxycilline trihydratée et du clavulanate de potassium ainsi que des excipients classiques, caractérisée en ce qu'elle contient un liant soluble dans l'eau non-hygroscopique en une quantité suffisante pour rendre cette composition extrudable après addition d'un solvant pour le liant.

2. Composition suivant la revendication 1, caractérisée en ce que le rapport pondéral d'amoxycilline à acide clavulanique est de 12:1 à 2:1.

3. Composition suivant la revendication 1 ou la revendication 2, caractérisée en ce que le rapport pondéral d'amoxycilline à acide clavulanique est de 8:1, 4:1 ou 2:1.

4. Composition suivant l'une quelconque des revendications 1 à 3, caractérisée en ce que le liant non-hygroscopique soluble dans l'eau est un polymère organique.

5. Composition suivant l'une quelconque des revendications 1 à 4, caractérisée en ce que le liant non-hygroscopique soluble dans l'eau est de l'hydroxypropylméthylcellulose, un copolymère de polyvinyl-pyrrolidone/acétate de polyvinyle ou de l'hydroxypropylcellulose.

6. Extrudat caractérisé en ce qu'il est obtenu à partir d'une composition suivant l'une quelconque des revendications 1 à 5.

7. Extrudat suivant la revendication 6, caractérisé en ce que le poids d'extrudat dans une dose unique est entre 0,25 et 10 g.

8. Extrudat suivant la revendication 6, caractérisé en ce que le poids d'extrudat dans une dose unique est entre 0,5 et 5 g.

9. Procédé de préparation d'un extrudat suivant l'une quelconque des revendications 6 à 8, caractérisé en ce qu'on associe de l'amoxycilline trihydratée, du clavulanate de potassium, un liant soluble dans l'eau, non-hygroscopique et des excipients classiques dans un solvant pour ce liant et qu'ensuite, on extrude le mélange résultant.

10. Composition utile dans le traitement d'infections bactériennes chez l'homme, caractérisée en ce qu'elle comprend de l'amoxycilline trihydratée et du clavulanate de potassium sous forme d'un extrudat suivant l'une quelconque des revendications 6 à 8.